# EUROPEAN PATENT SPECIFICATION

(11) **EP 2 260 014 B1**
(45) Date of publication and mention of the grant of the patent: **28.09.2011**
(21) Application number: 09725255.5
(22) Date of filing: 26.03.2009
(51) Int. Cl.: C07C 1/32, C07C 13/04

(54) **PROCESS FOR THE SYNTHESIS OF ETHYNYLCYCLOPROPANE**
VERFAHREN ZUR SYNTHESE VON ETHYNYLCYCLOPROPAN
PROCEDE POUR LA SYNTHESE D'ETHYNYLCYCLOPROPANE

(30) Priority: 26.03.2008 EP 08005556; 26.03.2008 US 64788 P
(43) Date of publication of application: 15.12.2010
(73) Proprietor: Lonza Ltd, 4052 Basel (CH); Lonza Guangzhou Research & Development Center Ltd, Guangdong Province 511455 (CN)
(72) Inventor: HANSELMANN, Paul, 3902 Brig-Glis (CH); WONG, Heilam, Guangdong 510275 (CN); CHEN, Yanlei, Foshan Guangdong 528313 (CN)
(74) Representative: Weber, Joachim
(86) International application number: PCT/EP2009/002206
(87) International publication number: WO 2009/118179

(56) References cited:
- EP-A- 0 922 686
- EP-A- 0 936 207

## Description

The present invention relates to a two step process for the preparation of ethynylcyclopropane of the formula 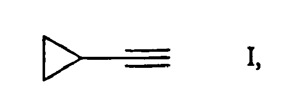 which is an intermediate in the synthesis of pharmaceutical active ingredients, for example of antiviral agents like (-)-6-chloro-4-cyclopropylethynyl-4-trifluoromethyl-1,4-dihydro-2*H*-3,1-benzoxazin-2-one, a potent HIV reverse transcriptase inhibitor (WO-A 96/22955).

The synthesis of the compound of formula I as disclosed in US-B-6552239 starts from cyclopropanecarboxaldehyde and a diazophosphonate. This reaction is also known as Seyferth-Gilbert alkyne synthesis. Cyclopropanecarboxaldehyde is obtained from (1,1-dichloroethyl)cyclopropane by eliminating two molecules of hydrogen chloride. A disadvantage of this process is that the preparation of the geminal dichloride requires drastic conditions (e.g. PCl₅) and that at least 2 equivalents of halide waste per equivalent of product have to be disposed.

EP-A-0936207 and GB-A-2355724 both disclose a cyclopropane ring-closure strategy. A 1,3-dihalopropane is reacted with an acetylide derivative and the resulting pentyne is cyclized in the presence of an organometal compound, for example n-butyllithium. The compound of formula I then is obtained in one or two further steps. These processes starting from 1,3-dihalopropane also produce at least two equivalents of halide waste per equivalent of product. A further disadvantage in an industrial scale is the very low reaction temperature of about -70 to -100 °C.

In another process as disclosed in EP-A 922686 two equivalents of methanol are directly eliminated of (1,1-dimethoxyethenyl)cyclopropane.

US 6297410 discloses another approach wherein during the elimination enyne by-products are generated which are extremely difficult to separate from the product.

The process of US 6072094 involves an elimination step of a *cis-* and *trans*-vinyl thioether mixture. The preparation of said vinyl thioether requires a silylating agent. Depending on the *cis*/*trans*-balance of the starting material the product has to be separated from two different starting compounds. Since the final step is carried out with a *cis*/*trans*-isomeric mixture it involves both a *cis*- and a *trans*-elimination. However, the optimum reaction conditions for a *cis*-elimination are different from that of a *trans*-elimination.

The object of the present invention was thus to provide an alternative process for the preparation of ethynylcyclopropane which does not require very low temperatures and produces little or even no halide waste. Finally a process should be established wherein the final elimination step is carried out starting from a single compound and not from an isomeric mixture.

According to the invention, these objects are achieved by the following process.

Claimed is a process for the preparation of ethynylcyclopropane of the formula 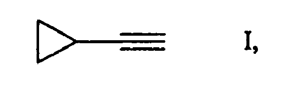 wherein in a first step (1,1-dimethoxyethyl)cyclopropane of the formula 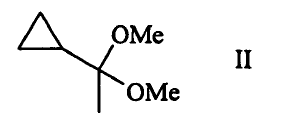 is reacted with a thiol of formula

HSR¹ III,

wherein R¹ is selected from the group consisting of C₁-C₆ alkyl, C₃-C₆ cycloalkyl, aryl and aralkyl wherein each alkyl, cycloalkyl, aryl and aralkyl can carry one or more halogen atoms, nitro, amino groups and/or a further substituent selected from the group consisting of C₁-C₆ alkyl C₁-C₆ alkoxy, and C₃-C₆ cycloalkyl, wherein such further substituent is optionally substituted with one or more halogen atoms, while two equivalents of methanol per equivalent of compound of formula II are eliminated, and a compound of formula 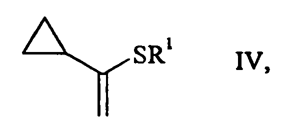 wherein R1 is as defined above, is obtained,
of which, in the second step, the thiol of formula III is eliminated to give the compound of formula I.

An advantage of the present process over the state of the art is that no halide waste is generated in the reaction.

Another advantage is that the second step of the present process can be carried out without heating the reaction mixture. Therefore the danger of formation of undesired by-products is reduced. Also, the instant process provides more than doubled overall yields (calculated on (1,1-dimethoxyethyl)-cyclopropane) as compared to EP-A 922686.

A further advantage is that the elimination step starts from a single starting compound. Therefore, the problem of isomeric mixtures is circumvented. Furthermore, the invention provides an efficient synthesis of ethynylcyclopropane with the use of inexpensive reagents.

Unlike the process disclosed in US 6072094 where a mixture of E- and Z-isomers of (2-cyclopropylvinyl)sulfanyl-benzene eliminates one equivalent of thiophenol to give ethynylcyclopropane in a moderate yield, the present (1-cyclopropylvinyl)sulfanyl-benzene provides a more sterically accessible proton and involves no *cis-* and *trans*-elimination. Therefore it gives a better yield.

The term "C₁-Cₙ alkyl", for example "C₁-C₁₈ alkyl", represents a linear or branched alkyl group having 1 to n carbon atoms. C₁-C₁₈ alkyl represents for example methyl, ethyl, propyl, isopropyl, butyl, isobutyl, *sec*-butyl, *tert*-butyl, pentyl, hexyl, octyl, decyl, dodecyl and octadecyl.

The term "C₁-Cₙ alkoxy", for example "C₁-C₆ alkoxy", represents a linear or branched alkoxy group having 1 to n carbon atoms. C₁-C₆ alkoxy represents for example methoxy, ethoxy, propoxy, isopropoxy, n-butoxy, isobutoxy, *sec*-butoxy, *tert*-butoxy, pentyloxy and hexyloxy.

The term "C₃-Cₙ cycloalkyl", for example "C₃-C₁₀ cycloalkyl", represents a cycloaliphatic group having 3 to n carbon atoms. C₃-C₁₀ cycloalkyl represents for example mono- and polycyclic ring systems such as cyclopropyl, cyclobutyl, cyclopentyl, cyclohexyl, cycloheptyl, cyclooctyl, adamantyl or norbornyl.

The term aryl represents an aromatic group, optionally substituted with one or more halogen atoms, amino groups, and/or optionally substituted C₁-C₆ alkyl, C₁-C₆ alkoxy or di-C₁-C₆ alkylamino groups. C₆-C₂₀ Aryl represents for example phenyl, naphthyl and derivatives thereof as outlined above.

The term aralkyl represents an aromatic group, wherein the alkyl moiety of the aralkyl residue is linear C₁-C₈ alkyl and the aryl moiety is selected from the group consisting of phenyl, naphthyl, furanyl, thienyl, benzo[b]furanyl, benzo[b]thienyl, optionally being substituted with one or more halogen atoms, amino groups, and/or optionally substituted C₁-C₆ alkyl, C₁-C₆ alkoxy or di-C₁-C₆ alkylamino groups.

Preferably, the first step of said process is carried out in the presence of at least one acidic catalyst. Furthermore, the acidic catalyst can be a solid acid or added as a salt like iron(III) chloride. Further preferred, said catalyst is a non-volatile inorganic or organic acid. Said organic acid can also be present in the form of its anhydride.

Suitable inorganic acids herein, include sulfuric acid, sulfurous acid, phosphoric acid, phosphorous acid, nitric acid, hydrobromic acid, hydrochloric acid, perchloric acid and boric acid, as well as Lewis acids such as include tin(II) chloride, tin(IV) chloride, tin(II) bromide, tin(IV) bromide, boron trifluoride, boron trifluoride etherate, trialkylaluminum, dialkyl-aluminum chlorides, alkylaluminum dichlorides, aluminum chloride, boron trichloride, boron tribromide, scandium bromide, scandium chloride, scandium iodide, titanium chloride, titanium bromide, iron(III) chloride, vanadium chlorides, chromium chlorides, manganese chlorides, zirconium chloride, zirconium bromide, zinc bromide, zinc chloride, yttrium chlorides, molybdenum chlorides, ruthenium chlorides and lanthanide chlorides such as lanthanum , cerium or ytterbium trichloride.

Suitable organic acids include alkanesulfonic acids such as methanesulfonic acid, butanesulfonic acid, (+, - or ±)-10-camphorsulfonic acid and arenesulfonic acids such as aminobenzene-2-sulfonic acid, 8-amino-naphthalene-1-sulfonic acid, benzenesulfonic acid, 2,5-diaminobenzenesulfonic acid, 5,6-bis(dimethylamino)-1-naphthalenesulfonic acid, 4-hydroxy-2-naphthalenesulfonic acid, 4-hydroxy-3-nitroso-1-naphthalenesulfonic acid tetrahydrate, 8-hydroxyquinoline-5-sulfonic acid, 1-naphthalenesulfonic acid, 2-naphthalenesulfonic acid, 1,5-naphthalenedisulfonic acid, 2,6-naphthalenedisulfonic acid, 2,7-napththalenedisulfonic acid, phenylboric acid, 2,4,6-trinitrobenzenesulfonic acid hydrate, 2-(2-pyridyl)ethanesulfonic acid, 2-(4-pyridyl)ethanesulfonic acid, 3-pyridinesulfonic acid, (2- hydroxypyridyl)methanesulfonic acid, sulfanilic acid, 2-sulfobenzoic acid hydrate, 5-sulfosalicylic acid hydrate, *p*-toluenesulfonic acid or 2,4-xylenesulfonic acid, as well as organic Lewis acids such as scandium trifluoromethansulfonate, silver trifluoromethanesulfonate, copper(I) trifluoromethanesulfonate and copper(II) trifluoromethanesulfonate.

An inorganic or organic solid acid can also be used as acidic catalyst. Suitable organic solid acidic catalysts include acidic ion exchange resins having sulfonic acid groups such as Amberlyst^{®} or Amberlite^{®}.
Suitable inorganic solid acidic catalysts can be selected from the group consisting of acidic aluminum oxides, acidic aluminosilicates and/or acidic silicates such as acidic zeolithes or montmorillonites, each suitable for heterogeneous catalysis.

The second step is preferably carried out in the presence of at least one equivalent of a strong base, preferably of at least two equivalents, more preferably of 2.5 to at 3 equivalents. Under basic conditions, ethynylcyclopropane most probably is initially produced in the deprotonated form as an acetylide, which, during aqueous workup, is protonated again to give the final product of formula I.

Suitable strong bases are organic "super bases" or alkali metal bases.

Organic super bases mean nitrogen containing polycyclic compounds having high proton affinities and basicities and contain extended π-systems, such as 1,5-diazabicycle[4.3.0]-non-5-ene (DBN) or 1,8-diaza-bicyclo[5.4.0]undec-7-ene (DBU).

Strong alkali metal bases in the meaning of the present process are for example alkali metal alkylides, wherein optionally the alkyl moiety has further substitutents; alkali metal C₁-C₆ alkylamides, wherein optionally the alkyl moiety has further substitutents; aryl alkali metal compounds, wherein optionally the aryl moiety has further substitutents; or alkali metal silazides, wherein optionally the silyl moiety has further substitutents. Examples of suitable alkali metal bases are methyllithium, ethyllithium, propyllithium, isopropyllithium, *n*-butyllithium, isobutyllithium, *tert*-butyllithium, pentyllithium, hexyllithium, lithium triphenylmethylide, phenyllithium, tolyllithium, lithium diisopropylamide (LDA), potassium diisopropylamide (KDA), sodium diisopropylamide (NDA), lithium 3-(aminopropyl)amide (LAPA), potassium 3-(aminopropyl)amide (KAPA), sodium 3-(aminopropyl)amide (NAPA), potassium bis(trimethylsilyl)-amide, lithium hexamethyldisilazide (LiHMDS), potassium hexamethyldisilazide (KHMDS) and sodium hexamethyldisilazide (NaHMDS).

Particularly preferred the strong base is selected from the group consisting of KAPA, LAPA, NAPA, LDA, KDA, NDA, LiHMDS, KHMDS and NaHMDS.
Most preferably the strong base is selected from the group consisting of KAPA, LAPA and NAPA.

The examples below illustrate how the process according to the invention is carried out, without limiting it thereto.

### Examples

### Example 1: (1,1-Dimethoxy-ethyl)-cyclopropane (II)

To a flask charged with cyclopropyl methyl ketone (50 g, 0.59 mol) was added trimethyl orthoformate (82 g, 0.77 mol), methanol (200 mL, 4.94 mol) and *p*-toluenesulfonic acid (0.25 g, 1.45 mmol) at 25 °C respectively. The solution was stirred at 25 °C for 2 h. Sodium methoxide (0.15 g, 2.78 mmol) were added to the solution under vigorous stirring. Methyl formate, methanol and trimethyl orthoformate were removed under reduced pressure (at about 250-300 mbar). The remainder was washed with brine (100 mL) and dried over Na₂SO₄ (15 g). (1,1-Dimethoxy-ethyl)-cyclopropane was obtained as a colorless liquid in 45-65%.

### Example 2: (1-Cyclopropylvinylsulfanyl)-benzene (IV)

To a flask fitted with an additional funnel was added (1,1-dimethoxy-ethyl)-cyclopropane (13 g, 0.10 mol), thiophenol (11 g, 0.10 mol), toluene (250 mL) and 10-camphorsulfonic acid (700 g, 3 mmol) respectively. A pressure-equalizing dropping funnel was charged with molecular sieve (beads) and fitted with a reflux condenser on top. The solution was refluxed at 130 °C (bath temperature) for 20 h. The mixture was washed with 10% NaOH aqueous solution (2x50 mL) and brine (100 mL) and dried over Na₂SO₄ (5 g). Toluene was removed under reduced pressure to give an oil containing 46% of 1-cyclopropylvinylsulfanyl)-benzene. The raw product can be further purified, for example by distillation.

### Example 3: Ethynylcyclopropane (I)

KAPA (potassium aminopropyl amide) was generated in situ either by potassium hydride and 1,3-diaminopropane (Brown, C. A. J., Org. Chem. 1978, 43, 3083-3084) or by potassium and 1,3-diaminopropane in the presence of a catalyst Fe(NO₃)₃ (Kimmel, T. et al. Org. Chem. 1984, 49, 2494-2496). (1-cyclopropylvinyl)sulfanyl)-benzene (358 mg, 2.03 mmol) was added drop wise to a flask charged with a freshly generated KAPA solution (10 mL, 1.27 M in 1,3-diaminopropane, 12.7 mmol) at 0 °C (bath temperature). The mixture was stirred at 25 °C for 3 h and then cooled to 0 °C. To the mixture was added ice water. Yield of ethynylcyclopropane in the raw product is 88%. The mixture was washed with 10% AcOH aqueous solution (2×50 mL) and brine (100 mL) and dried over Na₂SO4 (2 g). The raw product was purified by distillation. Bp.: 50-51 °C

## Claims

1. A process for the preparation of ethynylcyclopropane of the formula 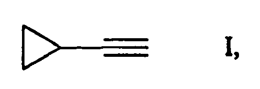 **characterized in that** in a first step (1,-1-dimethoxyethyl)cyclopropane of the formula 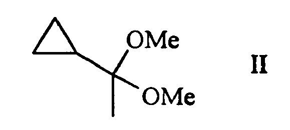 is reacted with a thiol of formula
HSR¹ III,
wherein R¹ is selected from the group consisting of C₁₋₆-alkyl, C₃₋₆-cycloalkyl, aryl and aralkyl wherein each alkyl, cycloalkyl, aryl and aralkyl can carry one or more halogen atoms, nitro, amino groups and/or a further substituent selected from the group consisting of C₁₋₆-alkyl C₁₋₆-alkoxy, and C₃₋₆-cycloalkyl, wherein such further substituent is optionally substituted with one or more halogen atoms, while two equivalents of methanol per equivalent of compound of formula II are eliminated, and a compound of formula 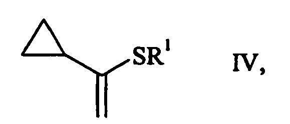 wherein R¹ is as defined above, is obtained,
of which, in the second step, the thiol of formula III is eliminated to give the compound of formula (I).

2. The process of claim 1, **characterized in that** the first step is carried out in the presence of an acidic catalyst.

3. The process of claim 2, **characterized in that** the catalyst is an inorganic or organic acid, or an anhydride thereof.

4. The process of any of claims 1 to 3, **characterized in that** the second step is carried out in the presence of at least two equivalents of a strong base, preferably of at least three equivalents, more preferably of at least six equivalents.

5. The process of claim 4, **characterized in that** the strong base is an organic super base or an alkali metal base.

6. The process of claim 5, **characterized in that** the organic super base is 1,5-diaza-bicyclo[4.3.0]non-5-ene (DBN) or 1,8-diaza-bicyclo[5.4.0]undec-7-ene (DBU).

7. The process of claim 5, **characterized in that** the alkali metal base is selected from the group consisting of optionally substituted alkali metal alkylides, optionally substituted alkali metal (C₁-C₆)alkylamides, aryl alkali metal compounds and alkali metal silazides.

8. The process of Claim 5, **characterized in that** the alkali metal base is selected from the group consisting of methyllithium, ethyllithium, propyllithium, isopropyllithium, n-butyllithium, isobutyllithium, *tert*-butyllithium, pentyllithium, hexyllithium, lithium triphenylmethylide, phenyllithium, tolyllithium, lithium diisopropylamide (LDA), potassium diisopropylamide (KDA), sodium diisopropylamide (NDA), lithium 3-(aminopropyl)amide (LAPA), potassium 3-(aminopropyl)amide (KAPA), sodium 3-(aminopropyl)amide (NAPA), potassium bis(trimethylsilyl)-amide, lithium hexamethyldisilazide (LiHMDS), potassium hexamethyldisilazide (KHMDS) and sodium hexamethyldisilazide (NaHMDS).

## Patentansprüche

1. Verfahren für die Herstellung von Ethinylcyclopropan der Formel 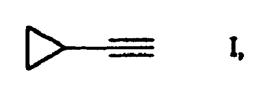 **dadurch gekennzeichnet, dass** in einem ersten Schritt (1,1-Dimethoxyethyl)cyclopropan der Formel 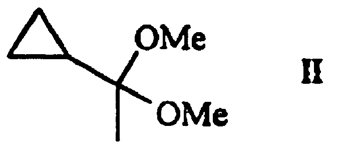 umgesetzt wird mit einem Thiol der Formel
HSR¹ III,
worin R¹ aus der Gruppe gewählt ist, die aus C₁-C₆-Alkyl, C₃-C₆-Cycloalkyl, Aryl und Aralkyl besteht, wobei jedes Alkyl, Cycloalkyl, Aryl und Aralkyl ein oder mehrere Halogenatome, Nitro-, Aminogruppen und/oder einen weiteren Substituenten, der aus der Gruppe gewählt ist, die aus C₁-C₆-Alkyl, C₁-C₆-Alkoxy und C₃-C₆-Cycloalkyl besteht, tragen kann, wobei ein solcher weiterer Substituent gegebenenfalls mit einem oder mehreren Halogenatomen substituiert ist, während zwei Äquivalente von Methanol pro Äquivalent der Verbindung der Formel II eliminiert werden, und eine Verbindung der Formel 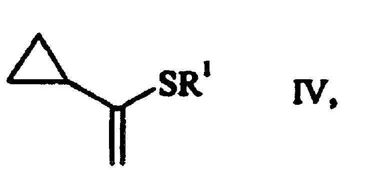 worin R¹ wie oben definiert ist, erhalten wird,
von welcher in dem zweiten Schritt das Thiol der Formel III eliminiert wird unter Erhalt der Verbindung der Formel (I).

2. Verfahren von Anspruch 1, **dadurch gekennzeichnet, dass** der erste Schritt in Gegenwart eines saure Katalysators durchgeführt wird.

3. Verfahren von Anspruch 2, **dadurch gekennzeichnet, dass** der Katalysator eine anorganische oder organische Säure oder ein Anhydrid davon ist.

4. Verfahren gemäß einem beliebigen der Ansprüche 1 bis 3, **dadurch gekennzeichnet, dass** der zweite Schritt in Gegenwart von mindestens zwei Äquivalenten einer starken Base, vorzugsweise von mindestens drei Äquivalenten, stärker bevorzugt von mindestens sechs Äquivalenten, durchgeführt wird.

5. Verfahren von Anspruch 4, **dadurch gekennzeichnet, dass** die starke Base eine organische Superbase oder eine Alkalimetallbase ist.

6. Verfahren von Anspruch 5, **dadurch gekennzeichnet, dass** die organische Superbase 1,5-Diaza-bicyclo[4.3.0]-non-5-en (DBN) oder 1,8-Diaza-bicyclo[5.4.0]-undec-7-en (DBU) ist.

7. Verfahren von Anspruch 5, **dadurch gekennzeichnet, dass** die Alkalimetallbase aus der Gruppe gewählt ist, die aus gegebenenfalls substituierten Alkalimetallalkyliden, gegebenenfalls substituierten Alkalimetall-(C₁-C₆-)alkylamiden, Arylalkalimetallverbindungen und Alkalimetallsilaziden besteht.

8. Verfahren von Anspruch 5, **dadurch gekennzeichnet, dass** die Alkalimetallbase aus der Gruppe gewählt ist, die aus Methyllithium, Ethyllithium, Propyllithium, Isopropyllithium, n-Butyllithium, Isobutyllithium, *tert*-Butyllithium, Pentyllithium, Hexyllithium, Lithiumtriphenylmethylid, Phenyllithium, Tolyllithium, Lithiumdiisopropylamid (LDA), Kaliumdiisopropylamid (KDA), Natriumdiisopropylamid (NDA), Lithium-3-(aminopropyl)amid (LAPA), Kalium-3-(aminopropyl)amid (KAPA), Natrium-3-(aminopropyl)amid (NAPA), Kalium-bis(trimethylsilyl)-amid, Lithiumhexamethyldisilazid (LiHMDS), Kaliumhexamethyldisilazid (KHMDS) und Natriumhexamethyldisilazid (NaHMDS) besteht.

## Revendications

1. Procédé de préparation d'éthynylcyclopropane de formule 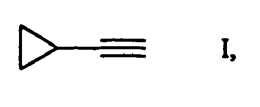 **caractérisé en ce que** lors d'une première étape du (1,1-diméthoxyéthyl)cyclopropane de formule 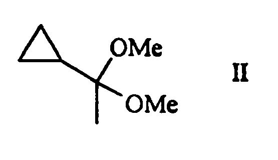 est mis en réaction avec un thiol de formule
HSR¹ III,
dans laquelle R¹ est choisi dans le groupe constitué par un alkyle en C₁₋₆, un cycloalkyle en C₃₋₆, un aryle et un aralkyle, chaque alkyle, cycloalkyle, aryle et aralkyle pouvant porter un ou plusieurs atomes d'halogène, groupes nitro, amino et/ou un autre substituant choisi dans le groupe constitué par un alkyle en C₁₋₆, un alcoxy en C₁₋₆ et un cycloalkyle en C₃₋₆, un tel autre substituant étant éventuellement substitué par un ou plusieurs atomes d'halogène, deux équivalents de méthanol par équivalent de composé de formule II étant éliminés et un composé de formule 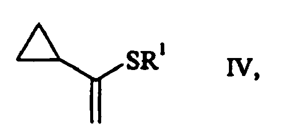 dans laquelle R¹ est tel que défini précédemment, étant obtenu, duquel, lors de la seconde étape, le thiol de formule III est éliminé pour obtenir le composé de formule (I).

2. Procédé selon la revendication 1, **caractérisé en ce que** la première étape est réalisée en présence d'un catalyseur acide.

3. Procédé selon la revendication 2, **caractérisé en ce que** le catalyseur est un acide inorganique ou organique, ou un anhydride de celui-ci.

4. Procédé selon l'une quelconque des revendications 1 à 3, **caractérisé en ce que** la seconde étape est réalisée en présence d'au moins deux équivalents d'une base forte, de préférence d'au moins trois équivalents, de manière davantage préférée d'au moins six équivalents.

5. Procédé selon la revendication 4, **caractérisé en ce que** la base forte est une super base organique ou une base de métal alcalin.

6. Procédé selon la revendication 5, **caractérisé en ce que** la super base organique est le 1,5-diaza-bicyclo[4.3.0]non-5-ène (DBN) ou le 1,8-diaza-bicyclo[5.4.0]undéc-7-ène (DBU).

7. Procédé selon la revendication 5, **caractérisé en ce que** la base de métal alcalin est choisie dans le groupe constitué par les alcalures de métaux alcalins éventuellement substitués, les alkylamides en C₁-C₆ de métaux alcalins éventuellement substitués, les composés aryliques de métaux alcalins et les silazides de métaux alcalins.

8. Procédé selon la revendication 5, **caractérisé en ce que** la base de métal alcalin est choisie dans le groupe constitué par le méthyllithium, l'éthyllithium, le propyllithium, l'isopropyllithium, le n-butyllithium, l'isobutyllithium, le tert-butyllithium, le pentyllithium, l'hexyllithium, le triphénylméthylure de lithium, le phényllithium, le tolyllithium, le diisopropylamide de lithium (LDA), le diisopropylamide de potassium (KDA), le diisopropylamide de sodium (NDA), le 3-(aminopropyl)amide de lithium (LAPA), le 3-(aminopropyl)amide de potassium (KAPA), le 3-(aminopropyl)amide de sodium (NAPA), le bis(triméthylsilyl)-amide de potassium, l'hexaméthyldisilazide de lithium (LiHMDS), l'hexaméthyldisilazide de potassium (KHMDS) et l'hexaméthyldisilazide de sodium (NaHMDS).
